# EUROPEAN PATENT APPLICATION

(11) **EP 4 425 182 A1**
(43) Date of publication of application: **04.09.2024**
(21) Application number: 22887048.1
(22) Date of filing: 26.10.2022
(51) Int. Cl.: G01N 33/68

(54) **METHOD FOR DETECTING KIDNEY DYSFUNCTION DIAGNOSTIC MARKER, METHOD FOR DETERMINING NEPHROPATHY PROGNOSIS, DETECTION KIT FOR KIDNEY DYSFUNCTION DIAGNOSTIC MARKER, AND KIDNEY DYSFUNCTION DIAGNOSTIC MARKER**

(30) Priority: 27.10.2021 US 202163272499 P
(71) Applicant: The University of Tokyo, Bunkyo-ku, Tokyo 113-8654 (JP); Japanese Foundation For Cancer Research, Tokyo 135-8550 (JP)
(72) Inventor: HARITA, Yutaka, Tokyo 113-8654 (JP); TAKIZAWA, Keiichi, Tokyo 113-8654 (JP); UEDA, Koji, Tokyo 135-8550 (JP)
(74) Representative: Nederlandsch Octrooibureau
(86) International application number: PCT/JP2022/039851
(87) International publication number: WO 2023/074723

(57) **Abstract**

A method for detecting a kidney dysfunction diagnostic marker, the method including measurement of an expression amount of MUC1 and/or MGAM in urinary extracellular vesicles derived from a subject.

## Description

### [Technical Field]

The present invention relates to a method for detecting a kidney dysfunction diagnostic marker, a method for determining a renal prognosis, a detection kit for a kidney dysfunction diagnostic marker, and a kidney dysfunction diagnostic marker.

### [Background Art]

Many pathologies can cause chronic kidney disease (CKD), but regardless of the cause, irreversible loss of functional nephrons is the foundation for development and progression of the disease. In recent years, childhood CKD has become a particular focus of research. This is because childhood CKD is responsible for increases in morbidity and mortality rates, and is also the cause of a variety of medical problems beyond childhood. Unlike adult CKD, non-glomerular kidney diseases including congenital anomalies of the kidney and urinary tract (CAKUT) and genetic disorders account for almost all cases of childhood CKD. Early diagnosis combined with therapeutic intervention offers significant benefits to both child and adult CKD patients.

However, with blood tests and urine tests, there is a possibility that the early stages of nephron loss may be overlooked. Identification of novel urinary biomarkers that enable better prediction of progression of the disease is required.

Extracellular vesicles (EV) are particles that are naturally released from cells separated by a lipid bilayer. Urinary EVs (uEV) or urinary exosomes contain specific proteins derived from the cells of all segments of the nephrons. These uEVs can function as urinary biomarkers resources that reflect fluctuations in molecule expression in the physiological or pathological states of the kidney. Accordingly, much uEV-based research of biomarkers for various diseases such as acute kidney injury, glomerular diseases, renal tubular disorders, polycystic kidney disease and renal transplantation has already been reported (for example, see Non-Patent Documents 1 and 2).

### [Citation List]

### [Non Patent Documents]

[Non Patent Document 1]
   Urinary extracellular vesicles: A position paper by the Urine Task Force of the International Society for Extracellular Vesicles. Erdbrugger U, et al., J Extracell Vesicles. 2021 May;10(7):e12093. doi: 10.1002/jev2.12093. Epub 2021 May 21.
[Non Patent Document 2]
   Extracellular Vesicles in Renal Diseases: More than Novel Biomarkers? Erdbrugger U, Le TH., J Am Soc Nephrol. 2016 Jan;27(1):12-26. doi: 10.1681/ASN.2015010074. Epub 2015 Aug 6.

### [Summary of Invention]

### [Technical Problem]

However, a biomarker for detecting quantitative change in functional nephrons, or a biomarker that uses uEVs has yet to be confirmed.

The present invention has been developed in light of the above circumstances, and provides a method for detecting a kidney dysfunction diagnostic marker, a method for determining a renal prognosis, a detection kit for a kidney dysfunction diagnostic marker, and a kidney dysfunction diagnostic marker that are capable of diagnosing kidney dysfunction accurately and non-invasively.

### [Solution to Problem]

The present invention includes the following aspects.
[1] A method for detecting a kidney dysfunction diagnostic marker, the method including measurement of the expression amount of MUC1 and/or MGAM in urinary extracellular vesicles derived from a subject.
[2] The method according to [1], wherein the urinary extracellular vesicles are phosphatidylserine-positive.
[3] The method according to [1] or [2], further including measurement of the expression amount of CD9 in the urinary extracellular vesicles.
[4] A method for determining a renal prognosis by calculating the ratio between the expression amount of MGAM and the expression amount of MUC1 in urinary extracellular vesicles derived from a subject, and determining a poor prognosis when the ratio is equal to or greater than a threshold.
[5] The method according to [4], wherein the urinary extracellular vesicles are phosphatidylserine-positive.
[6] A detection kit for a kidney dysfunction diagnostic marker, the kit containing an anti-MUC1 antibody and/or an anti-MGAM antibody.
[7] The kit according to [6], further containing a substance having affinity for urinary extracellular vesicles.
[8] The kit according to [7], wherein the substance having affinity is Tim4.
[9] A kidney dysfunction diagnostic marker, the marker including MUC1 and/or MGAM in urinary extracellular vesicles derived from a subject.
[10] The marker according to [9], wherein the urinary extracellular vesicles are phosphatidylserine-positive.
[11] The marker according to [9] or [10], also including CD9 in the urinary extracellular vesicles derived from a subject.

### [Advantageous Effects of Invention]

The present invention is able to provide a method for detecting a kidney dysfunction diagnostic marker, a method for determining a renal prognosis, a detection kit for a kidney dysfunction diagnostic marker, and a kidney dysfunction diagnostic marker that are capable of diagnosing kidney dysfunction accurately and non-invasively.

### [Brief Description of Drawings]

FIG. 1 is a schematic diagram outlining a discovery cohort and a validation cohort.
FIG. 2 is a diagram illustrating a scheme for separating uEVs from a urine sample using Tim4 affinity beads. The urine sample was centrifuged at 1,200 × g for 20 minutes at 4°C to remove cell debris and urine salts, and then centrifuged at 10,000 × g for 30 minutes at 4°C to remove the large EVs such as apoptotic bodies. Streptavidin magnetic beads bound with biotinylated Tim4 were added to the supernatant, and the resulting mixture was rotated for 1 hour at 20 to 25°C. The beads were then washed three times with a washing buffer, and the bound uEVs were eluted with an elution buffer.
FIG. 3 shows negative stain transmission electron microscope images of uEVs. The scale bars indicate 200 nm.
FIG. 4 illustrates the results of a tracking analysis of uEV nanoparticles isolated from healthy controls.
FIG. 5 is a Venn diagram of total protein detected in uEVs isolated from healthy controls.
FIG. 6 is a bar graph illustrating the abundance levels of typical classical exosome markers (CD63, CD9), a classical microvesicle marker (annexin A1) and an ARRDC1-mediated microvesicle marker (TSG101) in healthy controls. The y axis represents the log10 relative abundance.
FIG. 7 illustrates the results of analyzing the KEGG and Wiki pathways of 1,298 common proteins contained in the uEVs of healthy controls (g:Profiler). The 5 terms with the lowest adjusted P values were extracted.

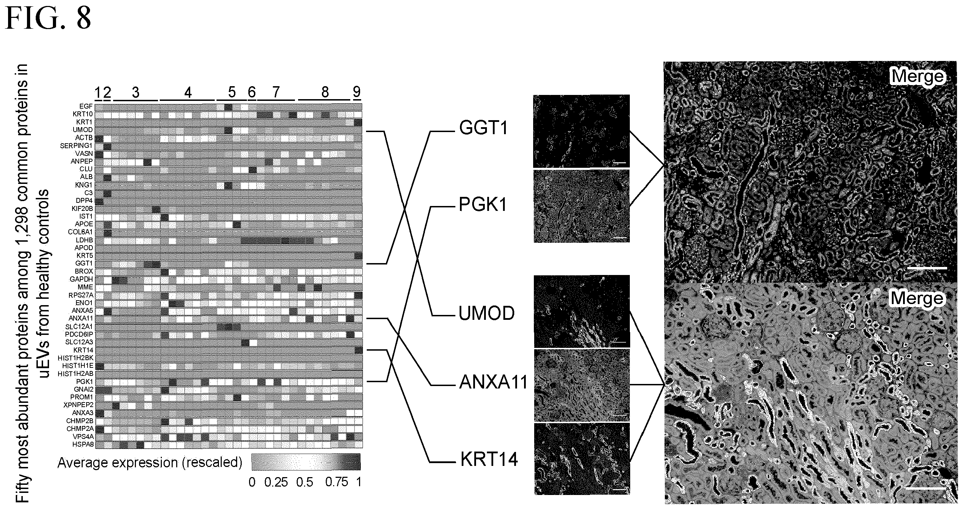

FIG. 8 is a diagram illustrating the relative abundance in each nephron fraction of the 50 most abundant molecules in uEVs from healthy controls visualized using Kidney Cell Explorer, and also showing an immunofluorescence image of a human kidney specimen for five of the 50 molecules. Glutathione hydrolase 1 proenzyme (GGT1), phosphoglycerate kinase 1 (PGK1), uromodulin (UMOD), annexin A11 (ANXA11) and keratin 14 (KRT14) are expressed in the proximal tubule, the loop of Henle, the distal tubule, the collecting duct, and the deep medullary epithelium of pelvis respectively. The numbers at the top of the drawing represent each nephron segment. 1: podocytes, 2: parietal epithelium, 3: proximal tubule, 4: loop of Henle, 5: distal tubule, 6: connecting tubule, 7: cortical collecting duct, 8: medullary collecting duct, 9: deep medullary epithelium of pelvis. The scale bars indicate 200 µm.
FIG. 9 is a table showing the baseline characteristics of participants in the discovery cohort.
FIG. 10 is a bar graph illustrating the abundance levels of typical classical exosome markers (CD63, CD9), a classical microvesicle marker (annexin A1) and an ARRDC1-mediated microvesicle marker (TSG101) in renal hypoplasia patients. The y axis represents the log 10 relative abundance.
FIG. 11 is a volcano plot displaying uEV proteins expressed in different amounts in healthy controls and renal hypoplasia patients. One hundred highly expressed proteins in healthy children, and 35 highly expressed proteins in renal hypoplasia patients are shown.
FIG. 12 illustrates a multidimensional scaling (MDS) analysis based on the quantitative profile of 135 proteins with characteristic expression changes.
FIG. 13 is a graph illustrating biological processes using gene ontology (GO) analysis(g:Profiler) of 35 proteins that exhibited increased expression and 100 proteins that exhibited reduced expression in the uEVs of renal hypoplasia patients.
FIG. 14 is a heat map of the expression of 135 proteins and the clinical information of 30 patients. The expression levels were subjected to logarithmic conversion and z-normalization to achieve a zero mean and unit standard deviation for each protein.
FIG. 15 is a series of violin plots illustrating examples of the expression of proteins in the uEVs of individuals with normal kidney function (N: eGFR ≥ 90 mL/min/1.73m²) and patients having reduced kidney function (D: eGFR < 90 mL/min/1.73m²). The data were compared using the two-sided Mann-Whitney U test. The label "ns" means not significant.
FIG. 16 illustrates a MDS analysis based on the quantitative profile of 135 proteins with characteristic expression changes.
FIG. 17 is a series of graphs illustrating the morphological characteristics of uEVs in CKD. (A) is a correlation plot of particle numbers and urinary creatinine. (B) is a correlation plot of particle size and particle numbers. (C) is a correlation plot of particle size and urinary creatinine. The correlation coefficients are shown as Pearson R coefficients.
FIG. 18(A) is a density plot illustrating the particle size distribution in healthy controls and CKD patients. The dotted lines indicate the mean size of the particles for each group. (B) is a box plot comparing the number of particles across the samples of healthy controls and CKD patients. (C) is a box plot comparing the mean size of the particles across the samples of healthy controls and CKD patients. (D) is a box plot comparing the peak size density across the samples of healthy controls and CKD patients. The data were compared using the two-sided Welch t-test.

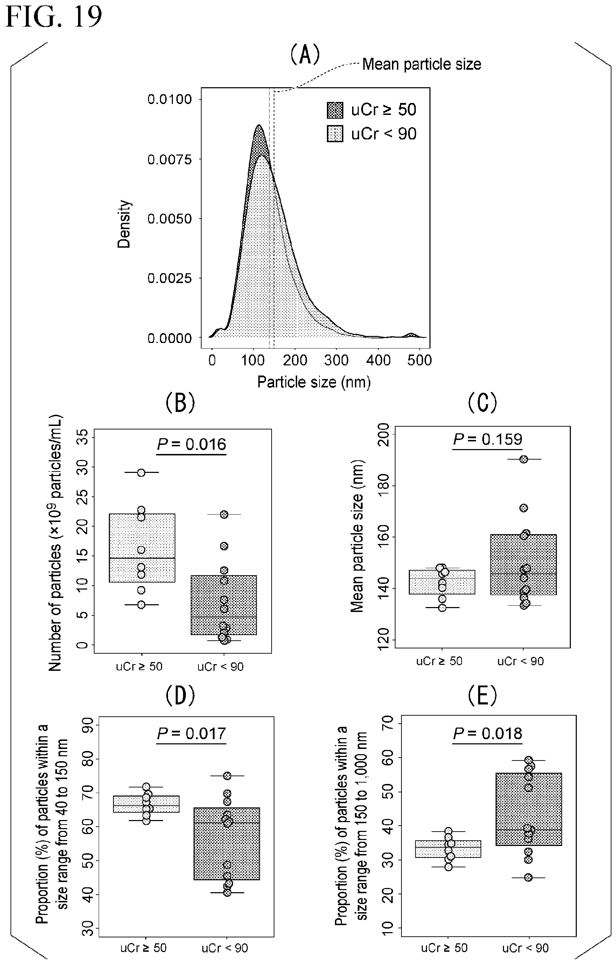

FIG. 19(A) is a density plot illustrating the particle size distribution in a sample in which uCr exceeded 50 mg/dL and a sample in which uCr was less than 50 mg/dL. The dotted lines indicate the mean size of the particles for each group. (B) is a box plot comparing the proportions of particle numbers. (C) is a box plot comparing the mean sizes of the particles. (D) is a box plot comparing the proportions of particles within a size range from 40 to 150 nm. (E) is a box plot comparing the proportions of particles within a size range from 150 to 1,000 nm. The data were compared using the two-sided Welch t-test.
FIG. 20 is a diagram illustrating the selection process for ELISA candidate molecules. Among the 35 molecules that increased in the uEVs of bilateral renal hypoplasia or CKD patients, MGAM was the only transmembrane protein. Among the 100 molecules with reduced expression in CKD, commercially available antibodies were used to evaluated 5 molecules. Of those, MUC1 was particularly suited to evaluation by Tim4-ELISA.
FIG. 21 illustrates the ELISA results for evaluating MUC1, MGAM and exosome markers (CD9 and CD63) in uEVs. The standard curves for MUC1, MGAM and the exosome markers (CD9 and CD63) were obtained by plotting the absorbance at 450 nm (y axis) against the value of the standard concentration (x axis). R² represents the correlation coefficient for the standard curve.

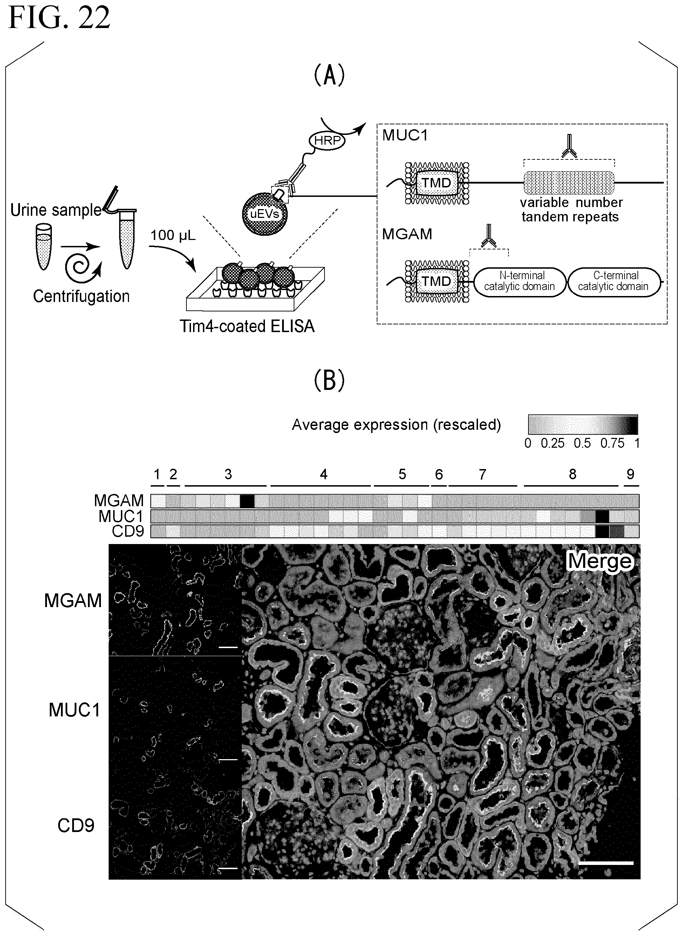

FIG. 22(A) is a schematic diagram of a sandwich ELISA evaluation using Tim4. (B) is an immunostaining image showing the expression of MGAM, MUC1 and CD9 in nephron segments. The expression patterns have been visualized by fluorescent immunohistochemical staining using Kidney Cell Explorer and human kidney tissue. The numbers at the top of the drawing represent each nephron segment. 1: podocytes, 2: parietal epithelium, 3: proximal tubule, 4: loop of Henle, 5: distal tubule, 6: connecting tubule, 7: cortical collecting duct, 8: medullary collecting duct, 9: deep medullary epithelium of pelvis. The scale bars indicate 100 µm.

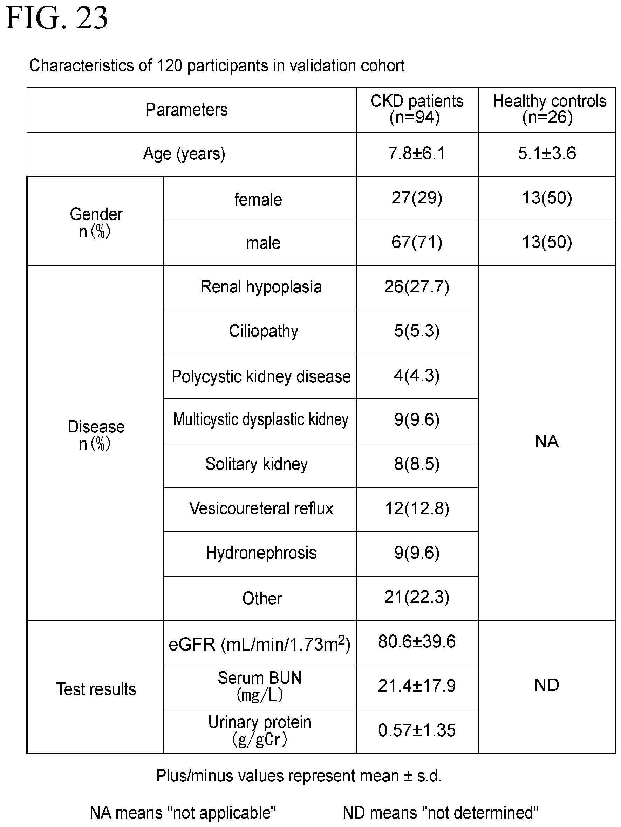

FIG. 23 is a table illustrating the characteristics of 120 participants in the validation cohort. The plus/minus values represent mean ± s.d. The term "other" includes horseshoe kidney, renal tubular dysgenesis, posterior urethral valve, ureterocele, henoch-schönlein purpura nephritis, nephrotic syndrome, asymptomatic proteinuria, obesity-related kidney disease, Epstein syndrome, nephrogenic diabetes insipidus, chronic kidney disease due to DOHaD, megaloureter, dysplastic kidney, nephrocalcinosis, and kidney damage caused by severe neonatal asphyxia. Further, "NA" represents "not applicable", and "ND" represents "not determined".
FIG. 24 is a series of graphs comparing the MUC1 level and MGAM level (absorbance at 450 nm), as measured by a customized ELISA, in samples of healthy controls and CKD patients. G1 indicates CKD patients in which eGFR ≥ 90, and G2-5 indicates CKD patients in which eGFR < 90 mL/min/1.73m².
FIG. 25(A) is an ROC curve for distinguishing patients having reduced kidney function (eGFR < 60) from healthy controls by logistic regression. (B) is an ROC curve for distinguishing patients having reduced kidney function (eGFR < 90) from healthy controls by logistic regression. (C) is an ROC curve for distinguishing CKD patients having a normal eGFR ≥ 90 from healthy controls by logistic regression.
FIG. 26 illustrates box and bee swarm plots of MGAM/MUC1 assay values (values obtained by dividing MGAM expression by MUC1 expression) in healthy controls and CKD patients having various kidney function in the discovery cohort and the validation cohort.
FIG. 27(A) is an ROC curve for distinguishing patients having reduced kidney function (eGFR < 60) from healthy controls by logistic regression using a combination of MGAM/MUC1, urinary creatinine (uCr), urinary albumin (uAlb) and L-FABP. (B) is a graph indicating the change in eGFR during a follow-up period in two groups (those having MGAM/MUC1 of less than 0.35, and others). The data were compared using the two-sided Mann-Whitney U test.
FIG. 28 is a set of graphs investigating the expression of exosome markers (CD9 and CD63) in the uEVs of the validation cohort. The CD9 level and CD63 level (absorbance at 450 nm) measured by a customized ELISA were compared in samples of healthy controls and CKD patients. G1 indicates CKD patients in which eGFR ≥ 90, and G2-5 indicates CKD patients in which eGFR < 90 mL/min/1.73m².
FIG. 29 is a series of graphs investigating the correlation between the ELISA expression levels of urinary albumin and MGAM, MUC1, CD9 and CD63.
FIG. 30 is a graph investigating the correlation between the MUC1 expression levels in uEVs and urine. The graph illustrates a correlation plot using 40 cases selected randomly from the validation cohort. The correlation coefficient is shown as a Pearson R coefficient. The x axis values indicate the absorbance at 450 nm by ELISA, and the y axis values indicate the urinary concentration (U/mL) of MUC1.

### [Description of Embodiments]

### <<Kidney Dysfunction Diagnostic Marker>>

This embodiment provides a kidney dysfunction diagnostic marker, wherein the marker includes MUC1 and/or MGAM in urinary extracellular vesicles derived from a subject.

Examples of kidney dysfunction, described in terms of diseases for which testing can be conducted, include chronic kidney disease, congenital anomalies of the kidney and urinary tract, renal hypoplasia, dysplastic kidney, renal tubular dysgenesis, hydronephrosis, solitary kidney, multicystic dysplastic kidney, nephronophthisis, ciliopathy, autosomal recessive polycystic kidney disease, autosomal dominant polycystic kidney disease, renal scarring, vesicoureteral reflux, megaloureter, fused kidney, and horseshoe kidney.

As described below in the examples, the inventors of the present invention comprehensively analyzed the proteins in uEVs isolated from renal hypoplasia patients, and discovered the above kidney dysfunction diagnostic marker.

There are no particular limitations on the test subjects, and examples include pediatric kidney disease patients and adult kidney disease patients.

MUC1 (Mucin-1) is a type I transmembrane glycoprotein belonging to the family of mucin proteins, and is a protein composed of an extracellular domain, a transmembrane domain, and a short cytoplasmic domain. The expression amount of this protein decreased in uEVs derived from renal hypoplasia patients.

MGAM (maltase-glucoamylase) is an α-glucosidase digestive enzyme, and is a transmembrane protein. The expression amount of this protein increased in uEVs derived from renal hypoplasia patients.

The urinary extracellular vesicles mentioned above are preferably phosphatidylserine-positive, and the kidney dysfunction diagnostic marker of this embodiment preferably also includes CD9. CD9 is a cell surface glycoprotein found on the exosome surface.

Examples of the kidney dysfunction diagnostic marker of this embodiment include MUC1; MGAM; and combinations of MUC1 and MGAM; MUC1 and CD9; MGAM and CD9; and MUC1, MGAM and CD9. The larger the number of marker molecules, the better the accuracy of the diagnosis.

### <<Detection Kit for Kidney Dysfunction Diagnostic Marker>>

This embodiment provides a detection kit for a kidney dysfunction diagnostic marker, wherein the kit contains an anti-MUC1 antibody and/or an anti-MGAM antibody. There are no particular limitations on these antibodies, provided they are capable of recognizing the antigen, and examples include monoclonal antibodies, polyclonal antibodies, multispecific antibodies (for example, bispecific antibodies), and antibody fragments.

The kit of this embodiment preferably also contains a substance having affinity for urinary extracellular vesicles. Examples of the substance having affinity for urinary extracellular vesicles include antibodies for molecules expressed on the urinary extracellular vesicle membrane such as CD9, and phosphatide-affinitive substances, and among these substances, phosphatidylserine-affinitive substances are preferred, and Tim4 is particularly desirable.

The kit of this embodiment preferably also contains a solid phase or carrier bound to the substance having affinity for urinary extracellular vesicle. Examples of the solid phase include a glass substrate, silicone substrate, plastic substrate, or metal substrate or the like, and the types of plastic substrates used in ELISA and the like are preferred.

Examples of the carrier include beads of silicon, titanium dioxide, aluminum oxide, glass, polystyrene, cellulose, or polyamide or the like.

The kit of this embodiment preferably contains a plastic substrate having immobilized Tim4, and an antibody for MUC1 and/or MGAM. In one example of a method for using the kit of this embodiment, the urinary extracellular vesicles from a subject are supplemented to a Tim4-immobilized plastic substrate, and a labelled antibody for MUC1 and/or MGAM is then used to detect the MUC1 and/or MGAM expressed at the surfaces of the urinary extracellular vesicles. Examples of the materials used for labelling the antibody for MUC1 and/or MGAM include marker enzymes, and specific examples include horseradish peroxidase and alkaline phosphatase.

### <<Method for Detecting Kidney Dysfunction Diagnostic Marker>>

This embodiment provides a method for detecting a kidney dysfunction diagnostic marker, wherein the method includes measurement of the expression amount of MUC1 and/or MGAM in urinary extracellular vesicles derived from a subject.

For example, the MUC1 protein expression amount in the urinary extracellular vesicles derived from a subject and a control expression amount from a control known to have a poor prognosis may be compared, and if the MUC1 protein expression amount in the subject is less than the control expression amount in the poor prognosis control, then the subject can be predicted as having kidney dysfunction.

Further, the MGAM protein expression amount in the urinary extracellular vesicles derived from a subject and a control expression amount from a control known to have a poor prognosis may be compared, and if the MGAM protein expression amount in the subject is greater than the control expression amount in the poor prognosis control, then the subject can be predicted as having kidney dysfunction.

The urinary extracellular vesicles are preferably phosphatidylserine-positive, and the method for detecting a kidney dysfunction diagnostic marker of this embodiment preferably also includes measurement of the expression amount of CD9 in the urinary extracellular vesicles. Measuring the expression amount of CD9 improves the accuracy of the diagnosis.

### <<Method for Determining Renal Prognosis>>

This embodiment provides a method for determining a renal prognosis, wherein the method includes calculating the ratio between the expression amount of MGAM and the expression amount of MUC1 in urinary extracellular vesicles derived from a subject, and determining a poor prognosis when the ratio is equal to or greater than a threshold.

As described below in the examples, it was confirmed that by setting the MGAM/MUC1 threshold to 0.35, the mean eGFR value at disease onset for patients having MGAM/MUC1 of 0.35 or higher was lower than that of the group for which MGAM/MUC1 was less than 0.35, and in some of the patients, the eGFR value decreased significantly. Further, the urinary extracellular vesicles are preferably phosphatidylserine-positive.

### [Examples]

The present invention is described below using a series of examples, but the present invention is not limited to the following examples.

### Outline of Human uEVs Isolated Using Tim4 Beads

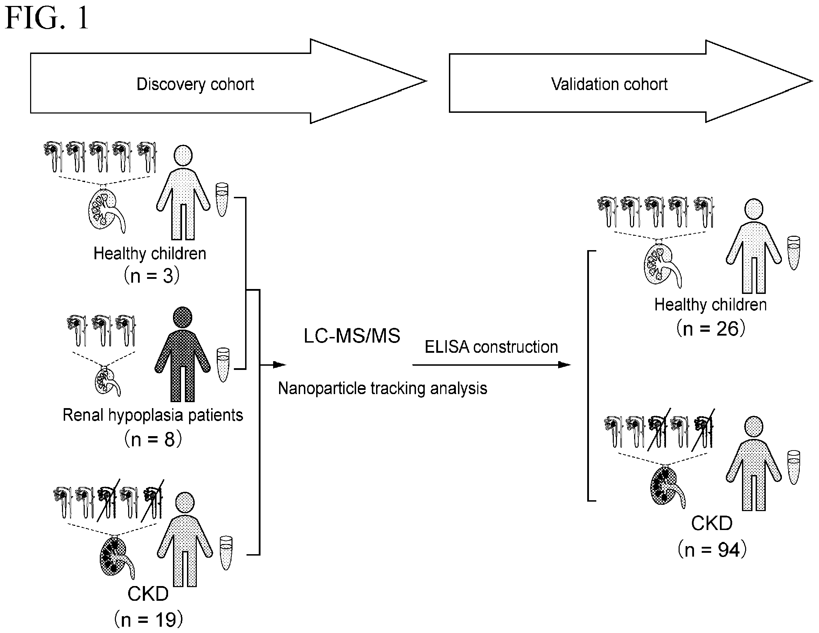

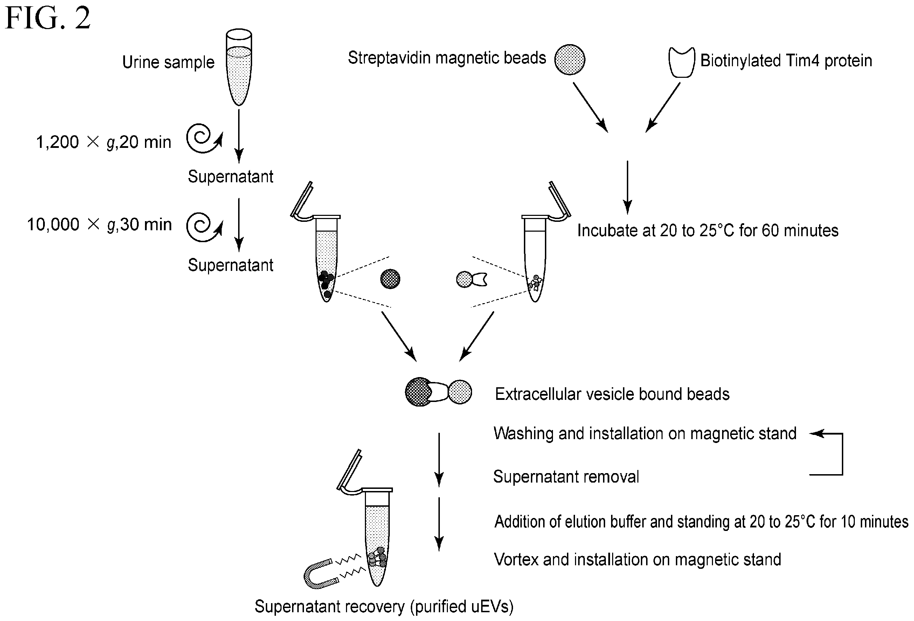

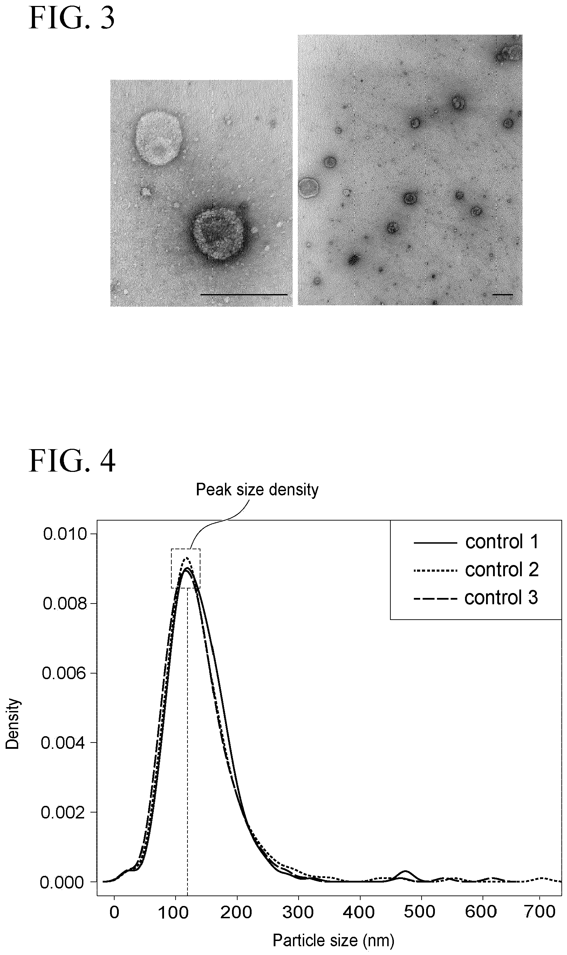

The protocol according to the present invention is illustrated in FIG. 1. In relation to the method used for isolating the uEVs, the differential ultracentrifugation method, which is the most widely employed technique, has a number of limitations. Because there is a possibility of damaging the EVs during the purification process, specialist technical expertise is required. There are also problems associated with the affinity purification method using exosome markers. Many uEVs do not express CD63 or CD9. This means that methods that use these molecules can suffer from bias in the vesicles purified from urine. Accordingly, the inventors of the present invention employed a Tim4-based purification system (see FIG. 2). The extracellular IgV-like domain of Tim4 binds to phosphatidylserine at the surfaces of EVs including exosomes and microvesicles. The binding is Ca²⁺ dependent, and therefore by adding a Ca²⁺ chelating agent, undamaged EVs can be easily released from the Tim4-bound base. The external appearance of the uEVs, which was clarified using a transmission electron microscope, matched the typical saucer-like shape (see FIG. 3).

The number and size distribution of the uEVs in the suspension were analyzed by nanoparticle tracking analysis (NTA). The mean number (±SD) of uEVs isolated from the control samples was 19.19 (±7.19) × 10⁹ particles/mL. The mean size (±SD) of the uEVs was 137.9 (±2.5) nm, and the peak was 117.47 nm (±1.0) nm (see FIG. 4).

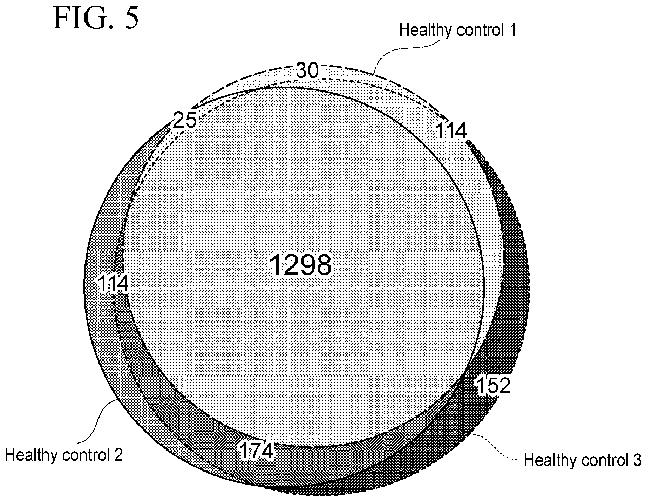

The protein composition of the uEVs isolated from the control samples was analyzed using liquid chromatography-tandem mass spectrometry (LC-MS/MS). The purified uEVs were analyzed individually using an LC-MS/MS system. Subsequent searching of the Sequest database identified 1,298 non-redundant proteins in all three control samples (see FIG. 5).

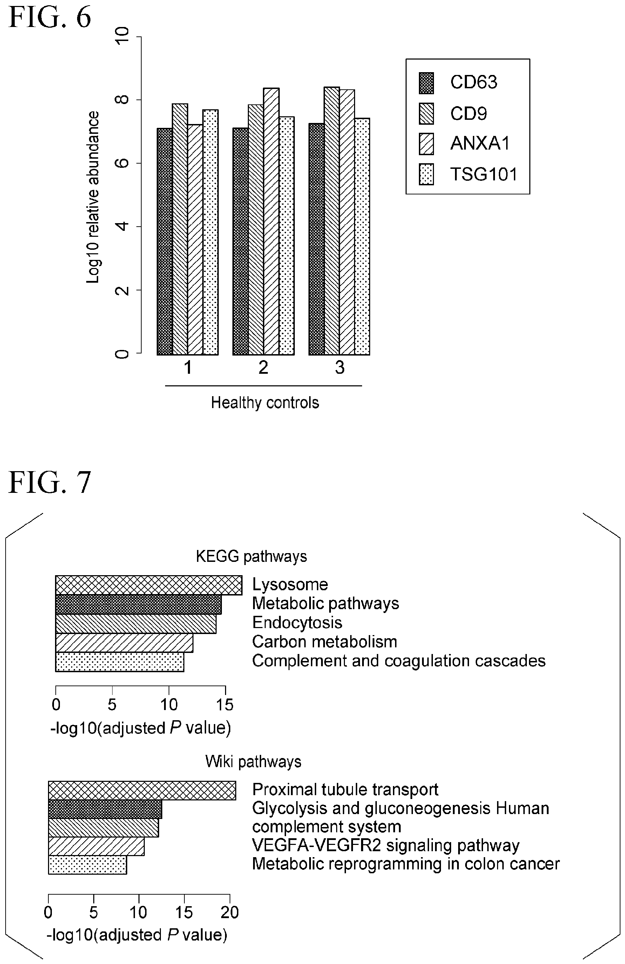

This list included almost all non-tissue-specific EV proteins (category 1a) and cytosolic proteins recovered in EVs (category 2). As expected, the typical exosome markers (CD63 and CD9), classical microvesicle marker (ANXA1), and the marker (TSG101) for ARRDC1-mediated microvesicles (ARMM) were detected in all three samples (see FIG. 6).

In the proteins contained in large amounts in the uEV samples, analysis using Kyoto Encyclopedia of Genes and Genomes (KEGG) and Wikipathways revealed a number of pathways enriched in the uEV proteome, including lysosomes, metabolic pathways, endocytosis, and proximal tubule transport (see FIG. 7). The heterogeneity of the cellular origins of the purified vesicles was confirmed by Kidney Cell Explorer, which visualizes the gene expression patterns in various cells of the nephrons, and also by immunofluorescence of human kidney specimens (see FIG. 8).

### uEV Changes in Renal Hypoplasia

Next, the inventors of the present invention focused their attention on uEVs isolated from bilateral renal hypoplasia patients. Renal hypoplasia is characterized by congenitally small kidneys having reduced numbers of nephrons. Renal scarring or renal atrophy arising from acquired diseases were excluded.

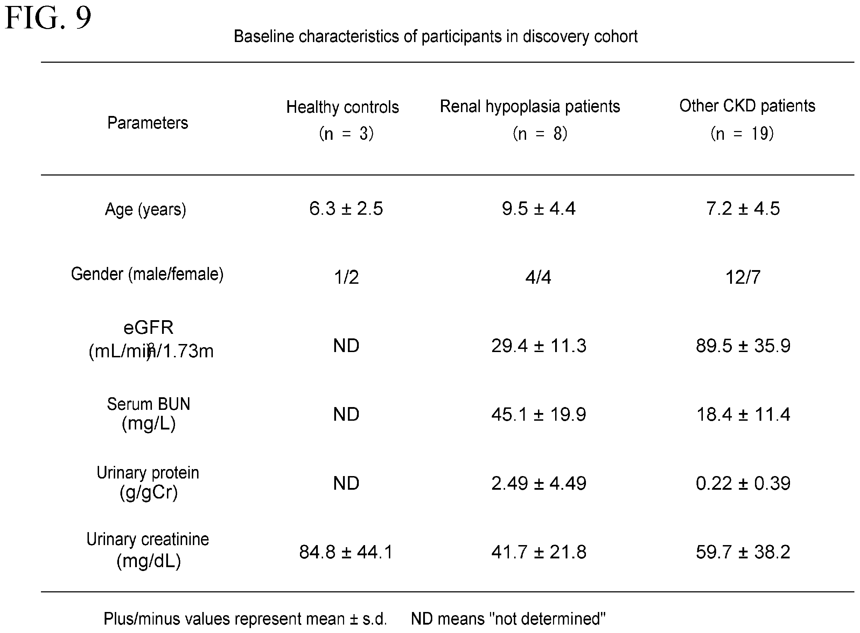

Characteristics of the patients are shown in FIG. 9. Following normalization for the total protein in each uEV sample, the protein compositions of the uEVs isolated from the renal hypoplasia patients were compared with those of the uEVs isolated from the control samples. Total protein is widely used as a normalizing variable when calculating the relative secretion rates of EVs.

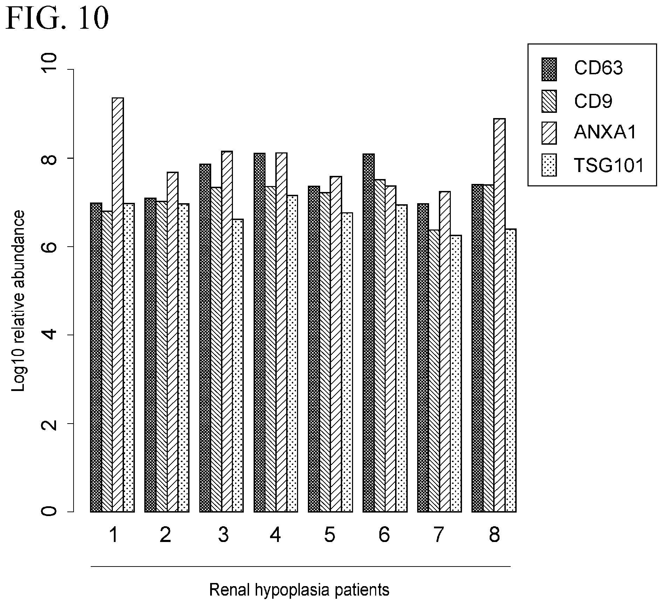

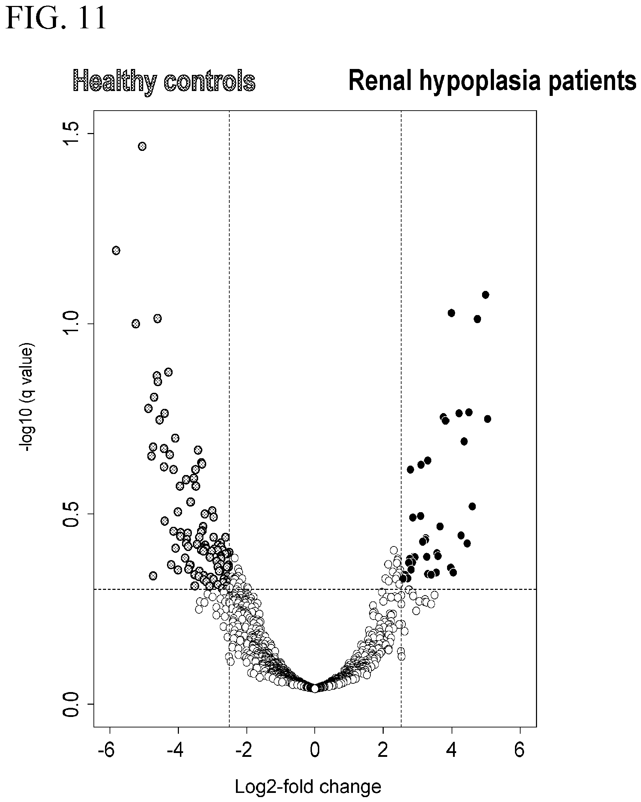

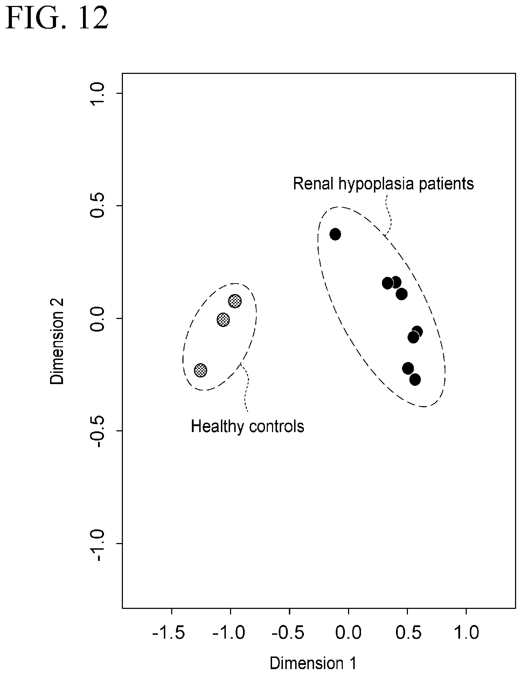

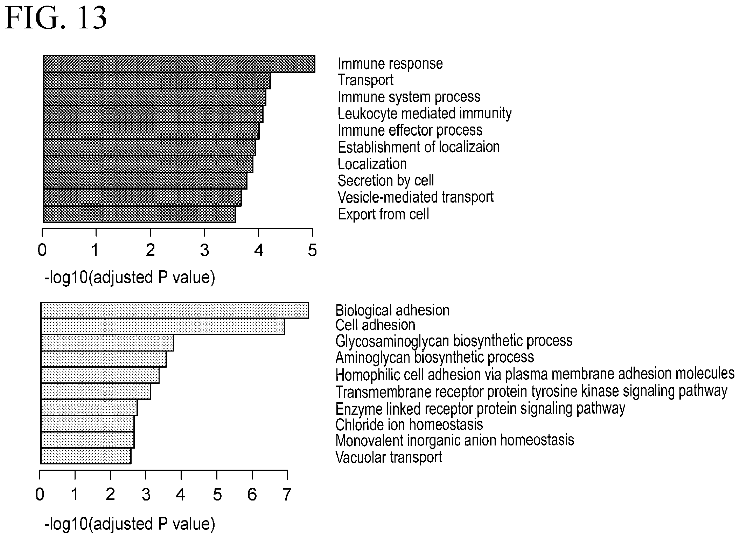

The uEVs from all of the renal hypoplasia patients contained marker proteins for classical exosomes (CD63 and CD9), classical microvesicles (ANXA1), and ARMM (TSG101) (see FIG. 10). Using enrichment analysis based on the protein-wise linear model in combination with empirical Bayesian statistics, the proteins that best distinguished the renal hypoplasia patients and the healthy controls were identified. The results revealed a total of 135 discriminatory proteins, and of those, 35 proteins increased and 100 proteins decreased with renal hypoplasia (see FIG. 11). Multidimensional scaling (MDS) analysis using the expression levels of these 135 proteins in the uEVs enabled the healthy controls and the renal hypoplasia patients to be distinguished (see FIG. 12). Gene ontology (GO) analysis confirmed that the proteins that exhibited an increased expression level with renal hypoplasia were associated with a number of immune-mediated pathways (see FIG. 13), and indicated the possibility of chronic kidney inflammation in CKD patients. On the other hand, the proteins that exhibited a reduced expression level with renal hypoplasia were associated with electrolyte transport in the renal tubules, such as chloride ion homeostasis and monovalent inorganic anion homeostasis (see FIG. 13).

### Expression Signatures and Characteristics of uEVs in CKD Patients

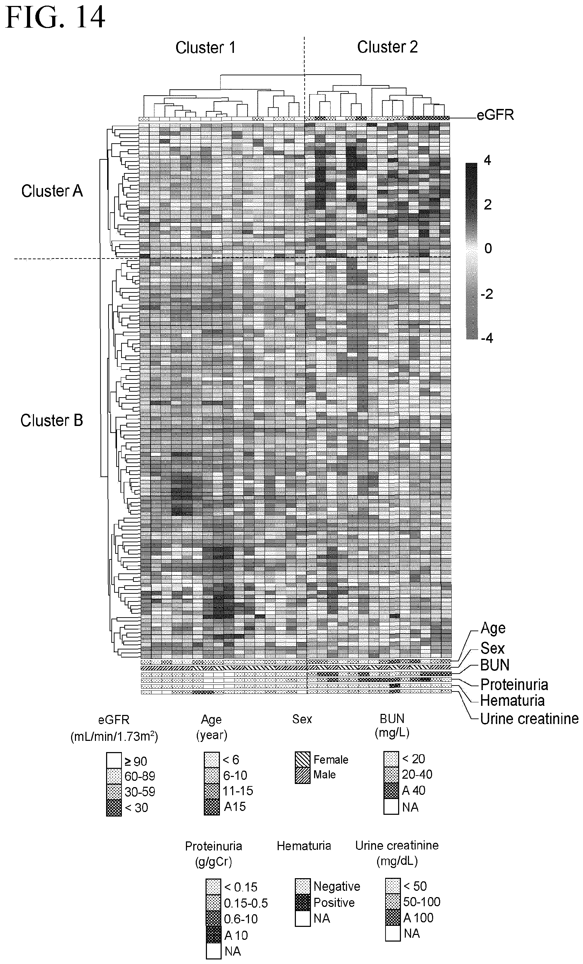

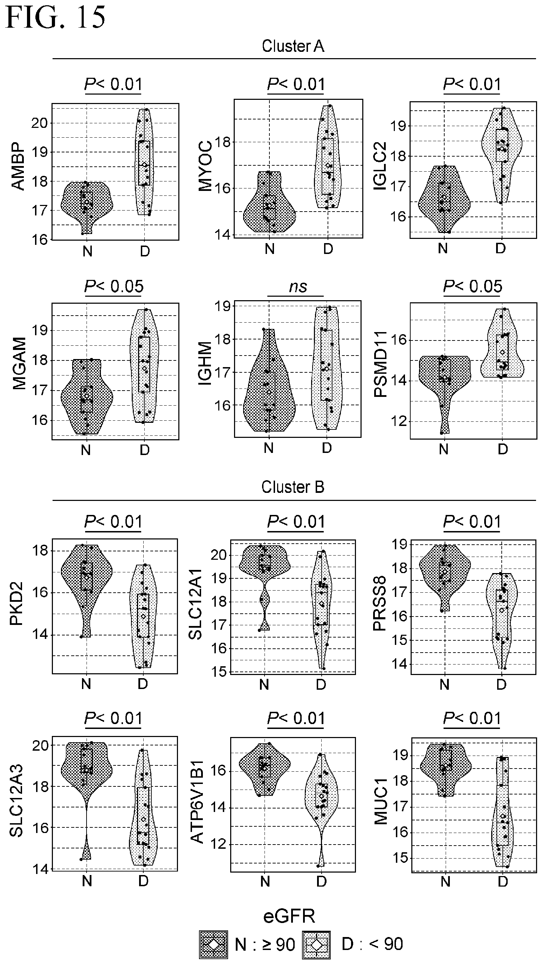

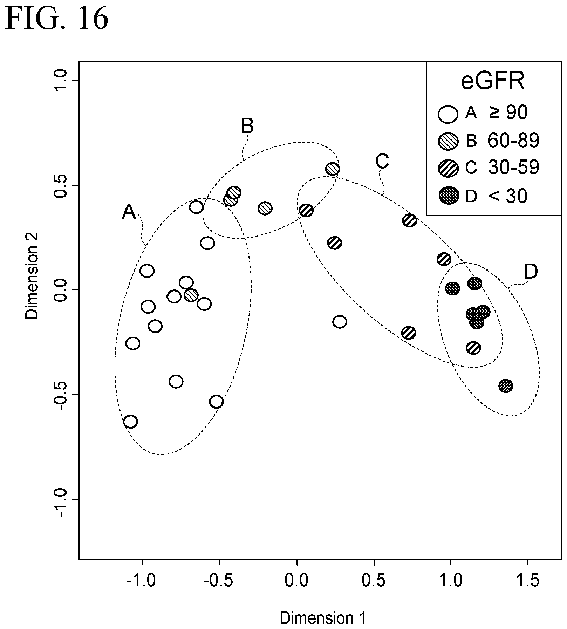

Next, samples were analyzed from CKD patients exhibiting a variety of kidney dysfunction due to congenital anomalies of the kidney and urinary tract (vesicoureteral reflux, solitary kidney, or unilateral renal hypoplasia with compensatory hypertrophy of the contralateral kidney) or ciliopathy (nephronophthisis or polycystic kidney disease) (see FIG. 9). These data were analyzed in combination with uEV data from the controls and bilateral renal hypoplasia patients. Using the quantitative expression data for the 135 proteins for distinguishing the controls and the bilateral renal hypoplasia patients, unsupervised consensus clustering of 30 uEV samples was conducted (see FIG. 14). This analysis revealed that these uEV samples could be classified into two main clusters (cluster 1 and cluster 2) (see FIG. 14). The mean eGFR of the patients of cluster 1 was 99.2 mL/min/1.73m², whereas the mean eGFR of the patients of cluster 2 had reduced significantly, and was 46.0 mL/min/1.73m². The abundance levels of representative proteins evaluated by LC-MS/MS analysis in those patients having an eGFR of at least 90 mL/min/1.73m² (N) and those patients having an eGFR of less than 90 mL/min/1.73m² (D) are illustrated in FIG. 15. MDS analysis using the quantitative proteome data demonstrated that the uEV proteome was able to distinguish those patients with reduced kidney function (see FIG. 16). These results suggest that the expression of these proteins could potentially be used in the urinary screening of patients with reduced kidney function.

### Characteristics of uEVs in CKD Patients

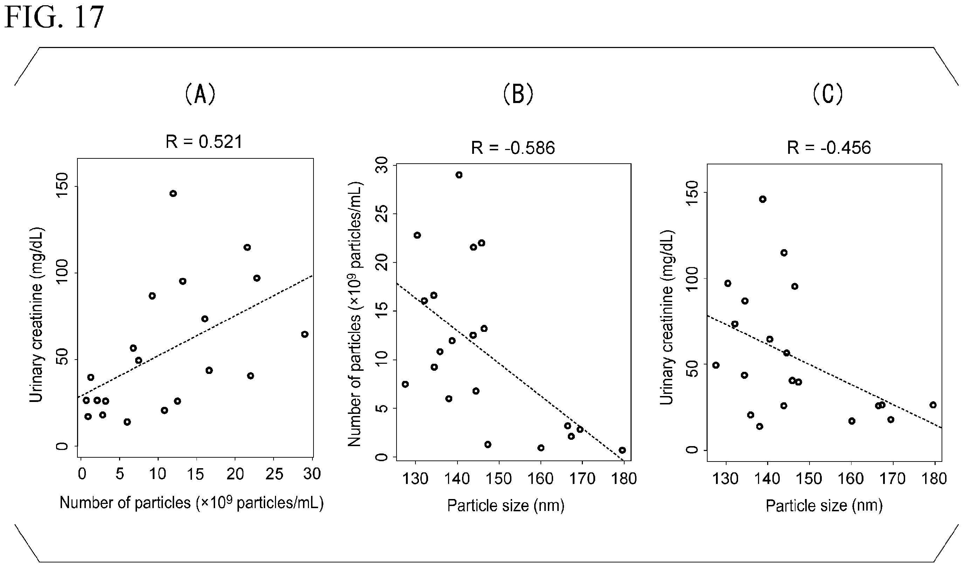

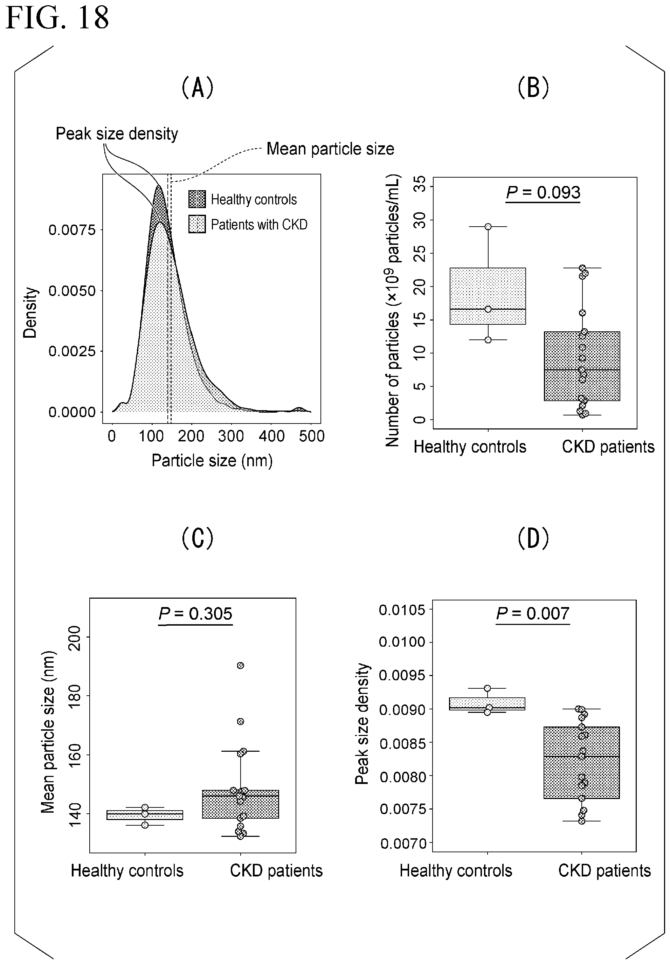

Next, an investigation was conducted to ascertain whether the physical characteristics of uEVs change with CKD. NTA was conducted using 20 samples having a sufficient sample size for investigation. The number of particles exhibited a positive correlation with urinary creatinine (see FIG. 17(A)), and a negative correlation with the mean size of the uEVs (see FIGS. 17(B) and (C)). Although the number of particles and the mean size of the uEVs from the CKD patients and the controls were not statistically different, the peak size was significantly lower in the CKD patients than in the controls (P=0.007) (see FIGS. 18(A) to (D)).

An investigation was conducted to ascertain whether the morphology of the uEVs changes with urinary creatinine. In the samples with low urinary creatinine (uCr < 50 mg/dL), the proportion of large vesicles (150 to 1,000 nm) was markedly higher than the samples with high urinary creatinine (uCr ≥ 50 mg/dL) (see FIG. 19).

### Construction of ELISA Platform for Detection of uEVs Signature

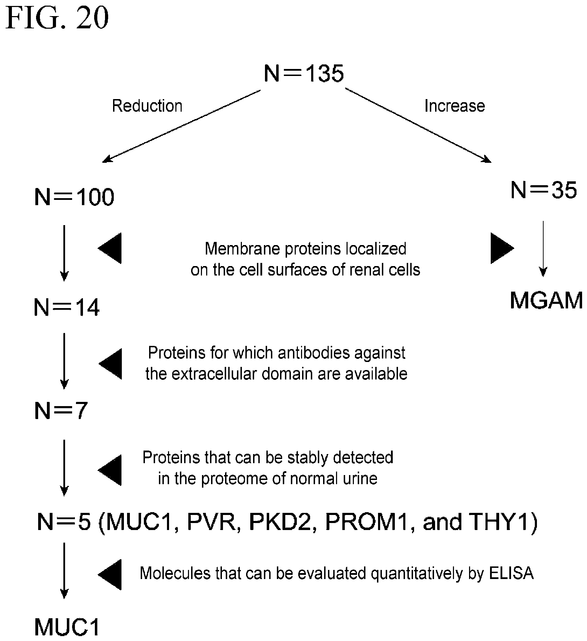

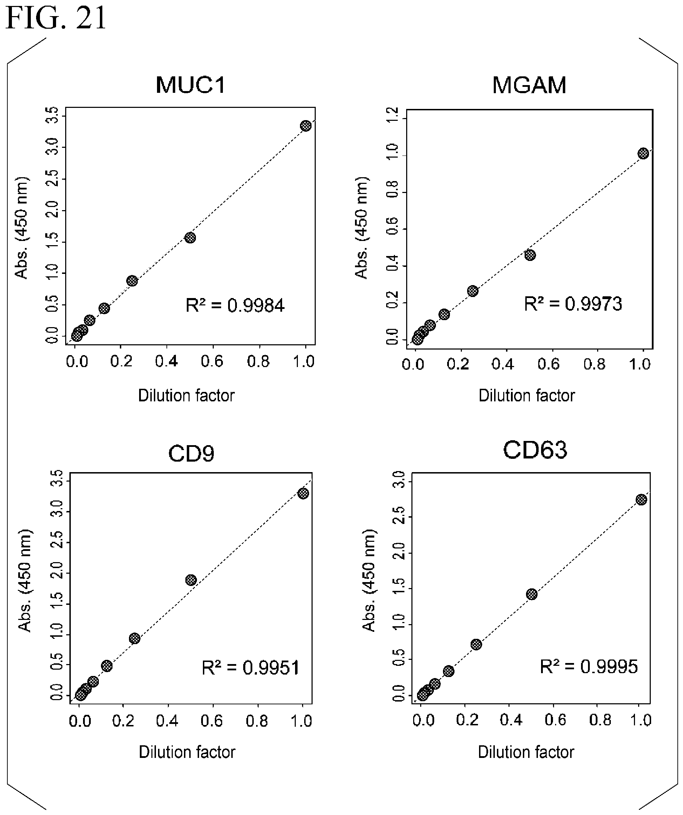

In order to develop proof of concept that the uEVs signature can be associated with CKD, an attempt was made to establish an ELISA platform using Tim4 as the EV-capturing substance, and biotinylated antibodies as the detection antibodies. The system is able to quantify the protein content of candidate biomarkers in the uEVs using a simple procedure. Among those molecules that exhibit increased expression in the uEVs of bilateral renal hypoplasia or CKD patients, MGAM was the only transmembrane protein having usable antibodies to the extracellular region (see FIG. 15). In order to narrow down the list of molecules that exhibit reduced expression in CKD, the following criteria were used: namely, a membrane protein that is localized on the cell surface of renal cells, has available antibodies that can be used against the extracellular region, and has a signal that can be stably detected in the proteome of normal urine. Five molecules (MUC1, PVR, PKD2, PROM1, and THY1) satisfied these criteria (see FIG. 15). Finally, an investigation was conducted as to whether commercially available antibodies could be used to evaluate the expression of these molecules in Tim4-purified uEVs. As a result of this analysis, the inventors finally succeeded in constructing a quantitative system for MUC1 and MGAM (see FIGS. 20 and 21).

MUC1 is a transmembrane glycoprotein, the expression of which is limited to the apical surfaces of distal tubules and collecting ducts. MGAM, which is an α-glucosidase, is expressed only in proximal tubular cells in the kidney (see FIG. 22(B)). Moreover, because the reduced particle size in patients with CKD corresponds with the size of the classical or nonclassical exosomes (see FIG. 18(A)), CD9 and CD63 were also included as markers for these small EVs (see FIG. 21). CD9 is expressed mainly at the basal membrane of the distal tubule and the collecting duct (see FIG. 22(B)).

### Utility of uEVs for Detection of CKD

Using a discovery cohort, MUC1 concentrations were ascertained by ELISA.

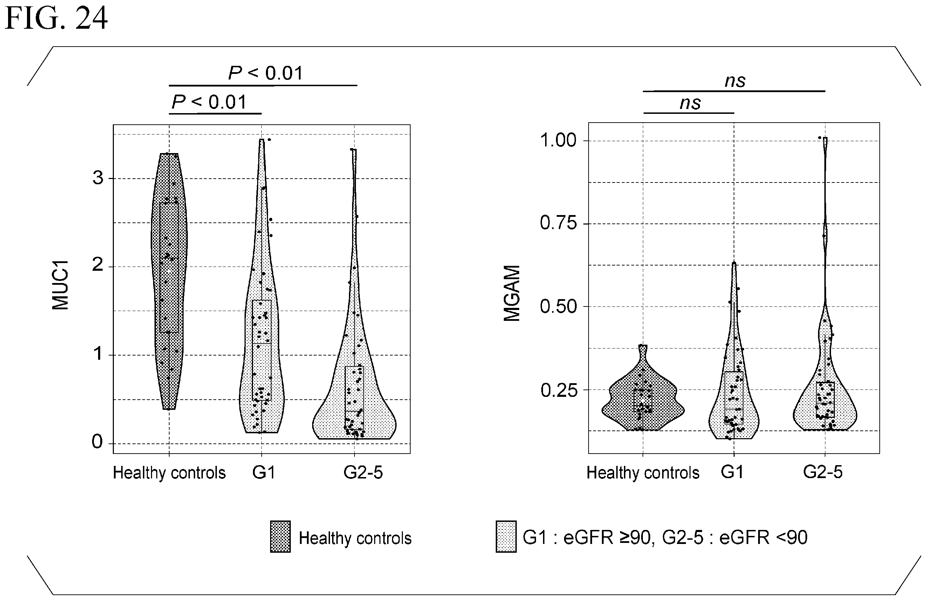

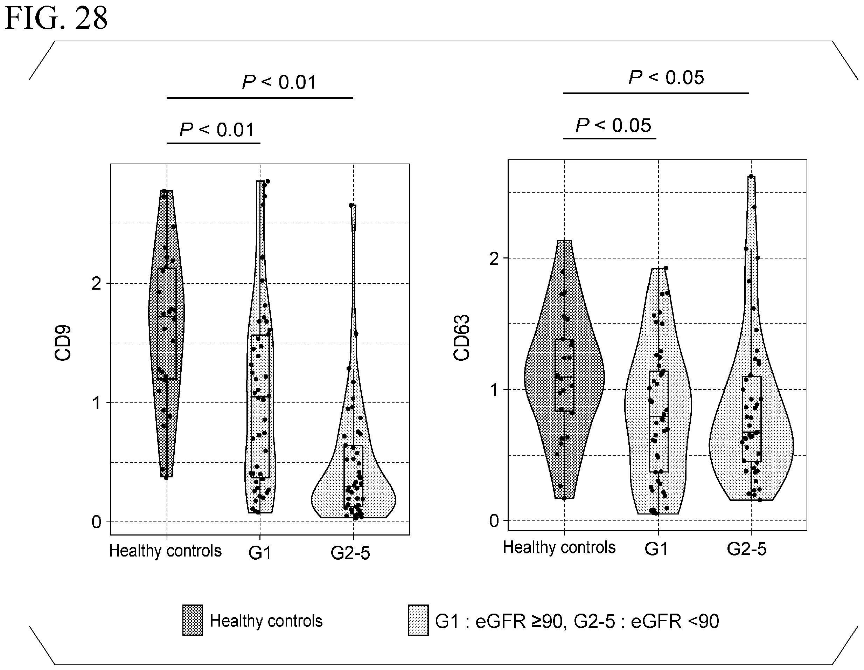

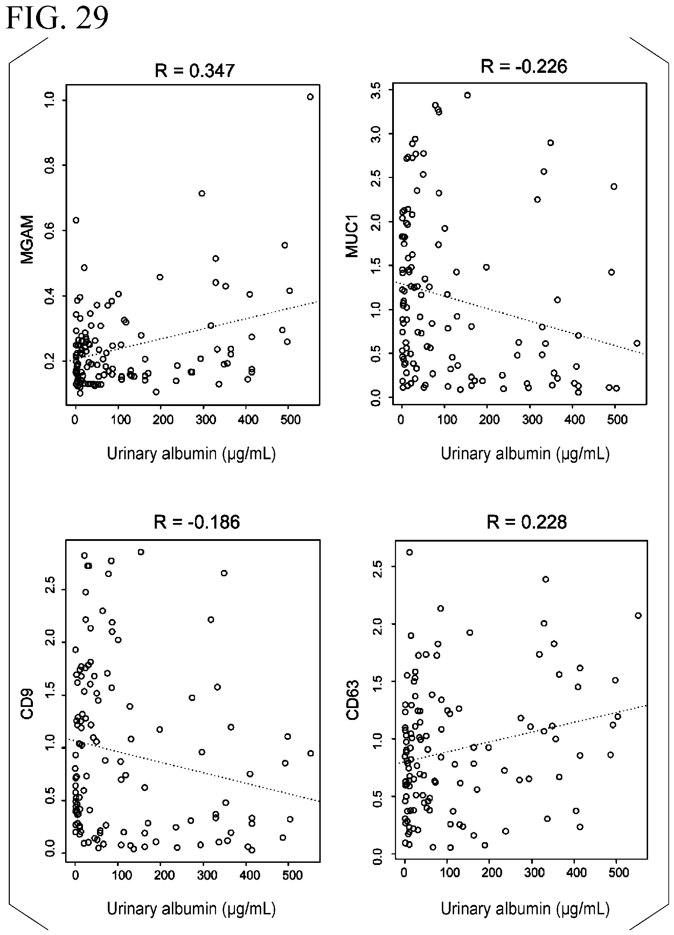

The sample size required for identification of reduced renal function of eGFR <60 or <90 by MUC1 was calculated as 10.8 or 23.9 respectively. Urine samples were collected from 26 controls and 94 pediatric patients with CKD (the validation cohort: see FIG. 23). Reflecting the etiology of childhood CKD, that patients suffered mainly from CAKUT, and no patients had diabetes or diabetic kidney disease. ELISA was used to quantify the MUC1, MGAM, CD9 and CD63 levels in the uEVs (see FIGS. 24 and 28). The expression levels of MUC1, MGAM, CD9 and CD63 in the uEVs did not correlate with urinary albumin (see FIG. 29).

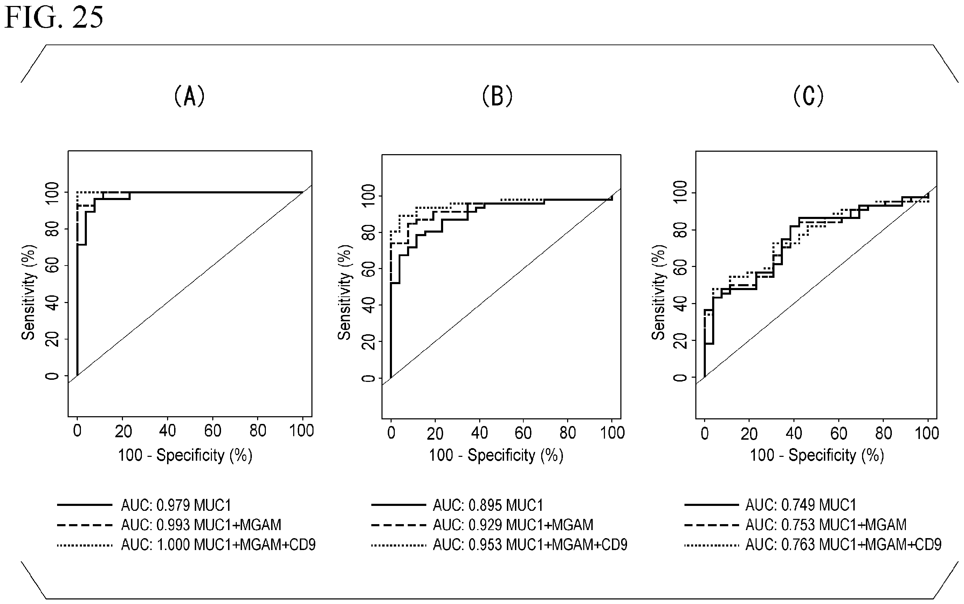

Receiver operating characteristic (ROC) curve analysis was performed to evaluate the discriminatory performance in identifying patients with reduced renal function. Univariate analysis using the MUC1 level in the uEVs showed high diagnostic performance for discriminating patients with eGFR < 60 (see FIG. 25(A)) or < 90 mL/min/1.73m² (see FIG. 25(B)).

The AUC values increased further in bivariate analysis with MUC1 and MGAM. Trivariate analysis using MUC1, MGAM and CD9 achieved the highest AUC values of 1.000 (eGFR < 60) (see FIG. 25(A)) and 0.953 (eGFR < 90) (see FIG. 25(B)). An investigation was also conducted as to whether this method could distinguish patients with CKD having a normal eGFR (eGFR ≥ 90) from healthy controls. The AUC values of the bivariate or trivariate analysis exceeded 0.75 (see FIG. 25(C)). This suggests that the uEV signature may be capable of detecting changes in the kidneys even in CKD patients with normal eGFR.

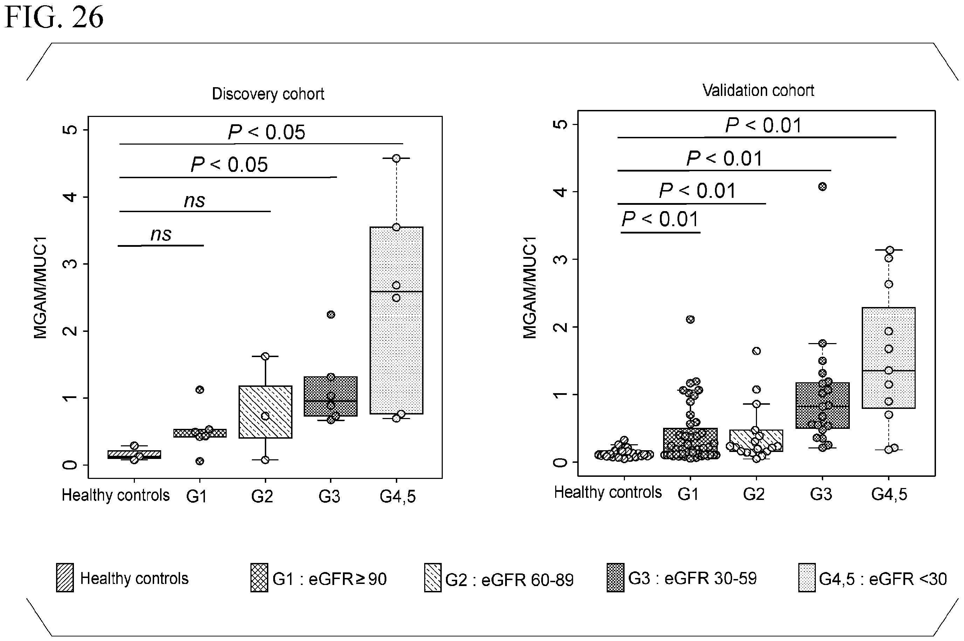

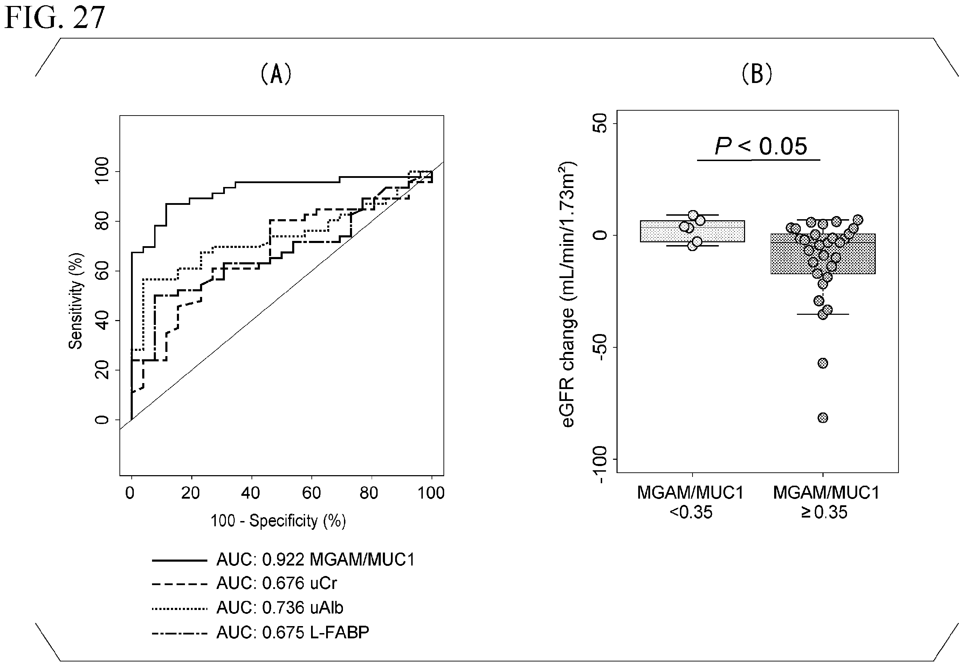

An investigation was also conducted as to whether the value of the expression value of MGAM divided by the expression value of MUC1 (MGAM/MUC1) could be used as an indicator for reduced kidney function instead of a combined multivariate analysis. The MGAM/MUC1 value increased as kidney function decreased in the discovery and validation cohorts (see FIG. 26). The MGAM/MUC1 value yielded an AUC of 0.922 for isolating patients with reduced kidney function (eGFR < 90) (see FIG. 27(A)). The sensitivity and specificity of MGAM/MUC1 for the diagnosis of decreased eGFR, based on the optimal cutoff value derived from the ROC curve, were 88.5% and 87.5% respectively. The MGAM/MUC1 value produces a significantly higher accuracy than urinary creatinine, albumin or L-FABP, suggesting that this new method of the present invention may capture changes that existing urine biomarkers are unable to detect (see FIG. 27(A)).

Finally, the association between MGAM/MUC1 and renal prognosis was analyzed. Among the patients in the discovery and validation cohorts, follow-up data for at least six months following the initial analysis could be used for 35 patients. These patients were classified into two groups based on the MGAM/MUC1 values in the initial analysis. The threshold (0.35) was set so as to include the maximum value (0.330) for MGAM/MUC1 among the healthy controls. The mean period from the initial analysis to the follow-up analysis was 25.8 (±14.4) months. In patients in which MGAM/MUC1 was less than 0.35 (<0.35, 6 patients), the mean eGFR at disease onset was 86.7 (±26.7) mL/min/1.73m². In this group, eGFR did not change significantly during the follow-up period. The mean eGFR at disease onset for the patients in which MGAM/MUC1 was at least 0.35 (≥0.35, 29 patients) was 65.4 ± 36.2 mL/min/1.73m², considerably lower than the group for which MGAM/MUC1 < 0.35, and some of these patients experienced a significant further decline in eGFR (see FIG. 27(B)).

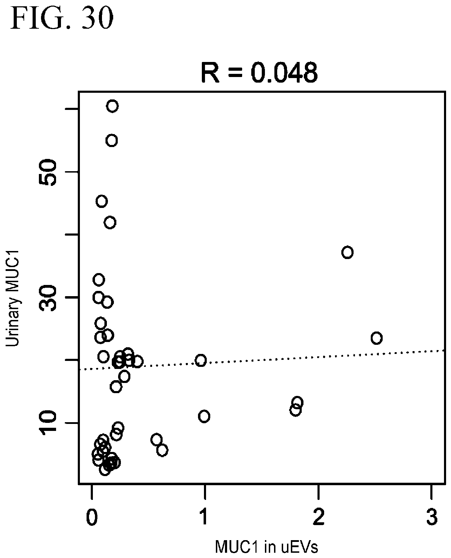

Urinary concentrations of MUC1 have been reported to be potentially associated with kidney disease. Patients with hypercalciuric nephrolithiasis had significantly decreased levels of urinary MUC1. Moreover, urinary MUC1 fragments that are shed from the renal tubular epithelium have been reported as a biomarker associated with kidney dysfunction. Accordingly, the inventors of the present invention investigated the correlation between the concentration of MUC1 in urine and in uEVs. As illustrated in FIG. 30, no correlation could be found between these two values. This result suggests that the change in MUC1 in uEVs captures changes in kidney tissue regardless of the urinary concentration of soluble MUC1 fragments.

### [Industrial Applicability]

Embodiments of the present invention enable kidney dysfunction to be diagnosed accurately and non-invasively.

## Claims

1. A method for detecting a kidney dysfunction diagnostic marker, the method comprising measurement of an expression amount of MUC1 and/or MGAM in urinary extracellular vesicles derived from a subject.

2. The method according to Claim 1, wherein the urinary extracellular vesicles are phosphatidylserine-positive.

3. The method according to Claim 1 or 2, further comprising measurement of an expression amount of CD9 in the urinary extracellular vesicles.

4. A method for determining a renal prognosis by calculating a ratio between an expression amount of MGAM and an expression amount of MUC1 in urinary extracellular vesicles derived from a subject, and determining a poor prognosis when the ratio is equal to or greater than a threshold.

5. The method according to Claim 4, wherein the urinary extracellular vesicles are phosphatidylserine-positive.

6. A detection kit for a kidney dysfunction diagnostic marker, the kit comprising an anti-MUC1 antibody and/or an anti-MGAM antibody.

7. The kit according to Claim 6, further comprising a substance having affinity for urinary extracellular vesicles.

8. The kit according to Claim 7, wherein the substance having affinity is Tim4.

9. A kidney dysfunction diagnostic marker, the marker including MUC1 and/or MGAM in urinary extracellular vesicles derived from a subject.

10. The marker according to Claim 9, wherein the urinary extracellular vesicles are phosphatidylserine-positive.

11. The marker according to Claim 9 or 10, also including CD9 in the urinary extracellular vesicles derived from a subject.
